# EUROPÄISCHE PATENTSCHRIFT

(11) **EP 1 008 582 B1**
(45) Veröffentlichungstag und Bekanntmachung des Hinweises auf die Patenterteilung: **23.04.2003**
(21) Anmeldenummer: 99122634.1
(22) Anmeldetag: 13.11.1999
(51) Int. Cl.: C07C 45/67, C07C 49/203

(54) **Verfahren zur Herstellung von ungesättigten Ketonen**
Process for the preparation of unsaturated ketones
Procédé de préparation de cétones insaturées

(30) Priorität: 07.12.1998 DE 19853908
(43) Veröffentlichungstag der Anmeldung: 14.06.2000
(73) Patentinhaber: BASF AKTIENGESELLSCHAFT, 67056 Ludwigshafen (DE)
(72) Erfinder: Oost, Carsten, Dr., 67098 Bad Dürkheim (DE); Stroezel, Manfred, 68549 Ilvesheim (DE); Etzrodt, Heinz, Dr., 67434 Neustadt (DE); Weller, Dietmar, Dr., 67067 Ludwigshafen (DE); Kaibel, Gerd, Dr., 68623 Lampertheim (DE); Jaedicke, Hagen, Dr., 67069 Ludwigshafen (DE)

(56) Entgegenhaltungen:
- EP-A- 0 842 917
- DE-A- 2 430 192
- DE-B- 1 286 018
- DE-B- 2 652 863

## Beschreibung

Die vorliegende Erfindung betrifft ein verbessertes Verfahren zur Herstellung von niederen ungesättigten Ketonen durch Umsetzen der entsprechenden α,β-ungesättigten Alkohole mit Acetessigsäurealkylestern in einer Carroll-Reaktion. Diese Reaktion ist, sieht man von den erfindungsgemäßen Verbesserungen ab, in ihren wesentlichen Merkmalen bereits bekannt.

Erstmals wurde eine derartige Umsetzung zwischen einem ungesättigten Alkohol und einem Acetessigsäurealkylester von Carroll in J. Chem. Soc. (London), 1940, Seiten 704 bis 706, beschrieben. Über den Anwendungsbereich und den Mechanismus dieser Reaktion wurde ein Jahr später von demselben Autor in J. Chem. Soc. (London), 1941, Seiten 507 bis 511, berichtet.

Aus DE 1 068 696 ist bekannt, daß 2-Methyl-2-hepten-6-on durch Zudosieren von 2-Methyl-3-buten-2-ol in ein auf 160 bis 180°C vorgewärmtes Reaktionsgemisch bestehend aus einem Acetessigsäurealkylester, einem Gemisch aus einem Acetessigsäurealkylester und einem inerten Lösungsmittel oder einem Gemisch aus 2-Methyl-3-buten-2-ol, einem Acetessigsäurealkylester und einem Lösungsmittel hergestellt werden kann. Die nach diesem Patent erzielten Ausbeuten von 66%, bezogen auf den eingesetzten Acetessigsäurealkylester, sind für ein technisches Verfahren völlig unzureichend.

Setzte man anstelle des Acetessigsäurealkylesters Diketen mit 2-Methyl-3-buten-2-ol in Gegenwart von Aluminiumtriisopropylat um, so erhielt man Ausbeuten von 83% (vgl. Advances in Organic Chemistry, Band II, 1960, Seite 246). Nachteilig an diesem Verfahren ist, daß die Unbeständigkeit von Diketen aus sicherheitstechnischen Gründen einen hohen apparativen Aufwand erfordert und daher hohe Investitions- und Betriebskosten für eine technische Anlage bedingt.

Es ist eine Reihe weiterer Patentschriften bekannt, in denen verschiedene Varianten der sogenannten Carroll'schen Reaktion beschrieben werden. So heißt es in US 2 795 617 (von 1957) bzw. DE AS 1 053 498 (von 1959) bzw. CH 342 947 (von 1959), daß "obschon es in der Regel weder notwendig noch erwünscht sei, ein Lösungsmittel verwendet werden kann, um den exothermen Reaktionsverlauf zu mildern". Gemäß den Verfahren dieser Patentschriften wurde das Aluminiumtrialkoholat zu dem Acetoacetat des α,β-ungesättigten Alkohols zugegeben und die Mischung unter starkem Rühren und unter Rückfluß zum Sieden erhitzt. Hierbei wurden Ausbeuten bis zu 80 % der Theorie erreicht. Nachteilig an diesem Verfahren ist, daß die Herstellung des als Ausgangsverbindung verwendeten Acetoacetats in einer gesonderten vorangehenden Stufe erfolgen muß.

DE 1 286 018 offenbart ein Verfahren zur Herstellung von 6,10-Dimethylundecatrien-(3,5,10)-on(2) durch Umsetzung von 3,7-Dimethyl-octen-(7)-in-(1)-ol-(3) mit Acetessigsäurealkylestern in Gegenwart eines sauren Katalysators. In US 2 839 579 (von 1958) bzw. DE 1 078 112 (von 1960) wird berichtet, daß die Umsetzung in einem Lösungsmittel durchgeführt werden kann. Die Herstellung des entsprechenden Acetoacetats erfolgt durch Kondensation des entsprechenden ungesättigten Alkohols mit Diketen in einer separaten Stufe.

Auch in DE 1 068 696 heißt es, daß die Mitverwendung eines Lösungsmittels vorteilhaft sein könnte. In allen Fällen werden hochsiedende Lösungsmittel genannt, deren Siedepunkte weit oberhalb der Reaktionstemperatur liegen.

Nachteilig an diesen Verfahren ist, daß die in diesen Patenten angegebenen Ausbeuten für eine technische Anwendung unbefriedigend sind und insbesondere, daß zur Herstellung des Acetoacetats des α,β-ungesättigten Alkohols eine zusätzliche Verfahrensstufe erforderlich ist, was zu zusätzlichen Kosten führt. Auch die vorgeschlagene Mitverwendung eines hochsiedenden Lösungsmittels bringt im allgemeinen keine nennenswerten Ausbeutesteigerungen mit sich und führt daher nur zu einer Reduzierung der Raumzeitausbeute. Ein Verfahren zur Herstellung von 2-Methyl-2-hepten-6-on wird in DE-AS 2 652 863 (von 1978) beschrieben. Hierbei werden der Acetessigsäurealkylester, 2-Methyl-2-buten-3-ol sowie der Katalysator in einem Reaktionsgefäß mit aufgesetzter Fraktionierkolonne vorgelegt und hierzu anschließend ein Gemisch aus dem Acetessigsäurealkylester und 2-Methyl-2-buten-3-ol zudosiert. Während der Reaktion soll der Gehalt an Acetessigsäurealkylester im Reaktionsgemisch nicht mehr als 15 Gew.-% betragen, um Nebenreaktionen zu vermeiden.

In dem Tschechischen Patent 216 360 (von 1979) wird empfohlen, Carroll-Reaktionen in einem Gemisch aus dem als Reaktionsprodukt zu erwartenden ungesättigten Keton und dem Acetessigsäuremethylester bzw. -ethylester unter Zugabe einer gerade zur Aufrechterhaltung der Reaktion erforderlichen Menge des ungesättigten Alkohols durchzuführen. Dabei wird aus dem Reaktionsgemisch das Kohlendioxid und ein Gemisch aus dem nicht umgesetzten ungesättigten Alkohol und Methanol bzw. Ethanol abdestilliert, welches kontinuierlich in einer angekoppelten Destillationskolonne fraktioniert wird. Der α,β-ungesättigte Alkohol, dessen Siedepunkt unter 180°C liegen muß, wird anschließend in die Reaktion zurückgeführt. Bei diesem Verfahren wurden bei Reaktionszeiten von 8 Stunden Ausbeuten von ca. 80 % der Theorie erreicht. Nachteilig an diesem Verfahren ist, daß durch die zusätzliche Destillationskolonne zusätzliche Investitions- und Energiekosten entstehen. Zudem sind die Ausbeuten und Reaktionszeiten bei diesem Verfahren für ein modernes technisches Verfahren unbefriedigend.

Weiterhin ist aus DE 2 928 944 (von 1979) die Herstellung von α,β-ungesättigten Ketonen durch Carroll-Reaktion unter Mitverwendung von kleinen Mengen eines Lösungsmittels beschrieben, dessen Siedepunkt zwischen dem des eingesetzten Acetessigsäurealkylesters und dem des hieraus abzuspaltenden Alkohols liegt. Dieses Lösungsmittel wird hierin als "Zwischensieder" bezeichnet. Als mögliche inerte Zwischensieder werden entsprechend siedende Alkohole, Ester, Ether, halogenierte Kohlenwasserstoffe und aromatische Kohlenwasserstoffe ,vorzugsweise aliphatische Ketone mit 4 bis 7 C-Atomen genannt. Als besonders vorteilhafte Ausführungsform wird die Verwendung von 2-Methyl-3-buten-2-ol als reaktivem Zwischensieder genannt, wobei als zusätzliche erwünschte Nebenreaktion dessen Umsetzung mit dem Acetessigsäurealkylester zu 2-Methyl-2-hepten-6-on als weiterem Wertprodukt stattfindet. Als Vorteile der Verwendung eines solchen Zwischensieders werden erhöhte Produktausbeuten (ca. 95 %der Theorie, bezogen auf den Alkohol, und ca. 85 % der Theorie, bezogen auf den Acetessigester), sowie kürzere Reaktionszeiten (ca. 4-5 h) und damit hohe Raumzeitausbeuten genannt. In allen Ausführungsbeispielen werden Reaktionstemperaturen von maximal 165°C angewendet.

Die Verwendung eines Zwischensieders bringt jedoch nicht nur Vorteile mit sich, sondern auch die folgenden Nachteile. So verringert sich beispielsweise bei Verwendung eines inerten Zwischensieders das für die Edukte zur Verfügung stehende Reaktorvolumen, d. h. die erzielbaren Raum-Zeit-Ausbeuten werden zwangsläufig kleiner. Außerdem führt beispielsweise die Mitverwendung eines reaktiven Zwischensieders, wie 2-Methyl-3-buten-2-ol, zu einer zwangsweisen Koppelung der Produktion von verschiedenen ungesättigten Ketonen, die aus vielerlei Gründen unerwünscht sein kann.

Es war daher die Aufgabe der Erfindung, die Umsetzung von relativ niedrig siedenden α,β-ungesättigten Alkoholen, mit Acetessigsäurealkylestern in einer Carroll-Reaktion zu den entsprechenden ungesättigten Ketonen so zu verbessern, daß man sie auch ohne Mitverwendung eines hochsiedenden Lösungsmittels und ohne eine Koppelung mit der Herstellung anderer ungesättigter Ketone durchführen kann. Dabei sollte im Vergleich zu den in der Literatur beschriebenen Synthesen zur separaten Herstellung der ungesättigten Ketone zumindest eine gleich gute, möglichst aber eine höhere Produktausbeute, bezogen auf den ungesättigten Alkohol und bezogen auf den Acetessigsäurealkylester bei kürzeren Reaktionszeiten erzielt werden. Insbesondere sollte das als Riechstoff und für die Herstellung anderer Riechstoffe begehrte Keton 2-Methyl-2-hepten-6-on ausgehend von 2-Methyl-3-buten-2-ol ohne die Nachteile des Standes der Technik mit höherer Selektivität und höherer Raumzeitausbeute hergestellt werden können.

Gegenstand der Erfindung ist dementsprechend ein Verfahren zur Herstellung von ungesättigten Ketonen der allgemeinen Formel I 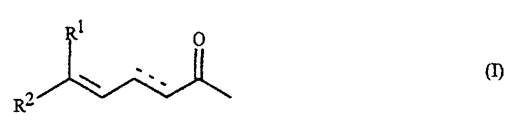 in der die gestrichelte Linie eine zusätzliche C-C-Bindung bedeuten kann, R¹ für eine Alkylgruppe mit 1 oder 2 C-Atomen steht und R² für eine Alkylgruppe mit 1 bis 4 C-Atomen steht, durch Umsetzen von α,β-ungesättigten Alkoholen der allgemeinen Formel II 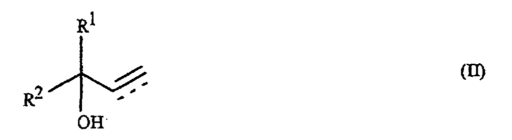 mit Acetessigsäurealkylestern der allgemeinen Formel III 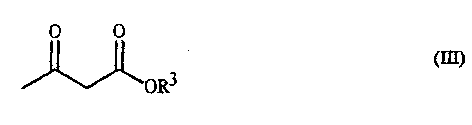 in der R³ für eine Alkylgruppe mit 1 bis 4 C-Atomen steht, in Gegenwart von 0.1 bis 5 Mol-%, bezogen auf den umzusetzenden Acetessigsäurealkylester, an einer organischen Aluminiumverbindung als Katalysator unter Abspaltung und fortlaufender destillativer Entfernung des sich während der Reaktion aus dem Acetessigsäurealkylester abspaltenden Alkohols der allgemeinen Formel IV

R³-OH (IV)

in einem Reaktorsystem mit aufgesetzter Fraktionierkolonne, das dadurch gekennzeichnet ist, daß man
- A: einen α,β-ungesättigten Alkohol, der unterhalb von 140°C siedet, insbesondere, 2-Methyl-3-buten-2-ol, ohne Mitverwendung wirksamer Mengen eines Lösungsmittels zusammen mit der organischen Aluminiumverbindung im Reaktionsgefäß vorlegt,
- B: unter erhöhtem Druck, eine möglichst konstante Reaktionstemperatur zwischen 170°C und 250°C, vorzugsweise zwischen 180°C und 200°C, einstellt,
- C: bei dieser Temperatur zu dem gemäß A erhaltenen Gemisch aus dem α,β-ungesättigten Alkohol und der organischen Aluminiumverbindung den Acetessigsäurealkylester zudosiert und
- D: während der Umsetzung den Gehalt an dem Acetessigsäurealkylester im Reaktionsgemisch auf einen möglichst konstanten Wert zwischen 0.1 und 10 Gew.-%, vorzugsweise zwischen 1 und 3 Gew.-%, einstellt.

Bei diesem Verfahren werden im Rahmen der Fehlergrenzen vergleichbare Selektivitäten (d.h. umsatzbezogene Ausbeuten, und nur diese werden in DE-AS 26 52 863 angegeben) an 2-Methyl-2-hepten-6-on ausgehend von 2-Methyl-3-buten-2-ol erzielt wie sie gemäß den Beispielen in DE-AS 26 52 863 erreicht wurden.

Die einsatzbezogenen Ausbeuten und der Umsatz, bezogen auf Methylbutenol, dagegen sind bei dem neuen Verfahren um ca. 10 Prozentpunkte höher als gemäß dem Verfahren gemäß DE-AS 26 52 863. Dies ist ein entscheidender Vorteil, da diese Ausgangsverbindung wesentlich teurer ist als der Acetessigsäurealkylester. Ein weiterer wesentlicher Vorteil ist, daß die höheren Umsätze bei gleicher Selektivität trotz einer um den Faktor 3 kleineren Reaktionszeit erzielt werden. Die Raumzeitausbeuten sind somit bei dem neuen Verfahren wesentlich höher, wodurch die Investitionskosten niedriger gehalten werden können.

Es war sehr überraschend, daß bei Anwendung der erfindungsgemäßen Bedingungen, d.h. insbesondere trotz Verzicht auf die Mitverwendung eines Lösungsmittels, Anwendung von höheren Drucken und Anwendung von Reaktionstemperaturen von 175 bis 220, vorzugsweise 180 bis 200°C, kaum Nebenreaktionen auftreten und dadurch ausgezeichnete Ausbeuten und Raumzeitausbeuten erhalten werden können. Es war weiterhin überraschend, daß sich mit Hilfe des erfindungsgemäßen Verfahrens selbst so niedrig siedende ungesättigte Alkohole, wie 2-Methyl-3-buten-2-ol, so vorteilhaft zu dem entsprechenden ungesättigten Keton, 2-Methyl-2-hepten-6-on, umsetzen lassen, da in DE-A-26 52 863 expressis verbis ausgeführt wird, daß ein einfaches Einführen des Acetessigsäurealkylesters in überschüssiges, die Aluminiumverbindung enthaltendes Methylbutenol bei der Reaktionstemperatur nicht möglich ist, da das 2-Methyl-3-buten-2-ol einen Siedepunkt von nur 98°C besitzt und die Umsetzung erst ab 140°C einsetzt. Es war auch nicht zu erwarten, daß trotz Verwendung höherer Drucke das aus dem Acetessigsäurealkylester gebildete Alkanol für die Beeinflussung der Reaktion ausreichend gut und schnell abdestilliert werden kann.

Mit dem beschriebenen Verfahren ergeben sich Produktausbeuten von ca. 95% der Theorie, bezogen auf den eingesetzten Alkohol. Die Selektivität der Umsetzung, d.h. die Ausbeute, bezogen auf den umgesetzten Alkohol, liegt sogar bei über 97% der Theorie, so daß bei einer möglichen Rückführung des nicht umgesetzten Alkohols Gesamtausbeuten von fast 100 % erreicht werden können. Die Produktausbeuten bezüglich des Acetessigsäurealkylesters liegen zwischen 90 % und 95 % bei vollständigem Abbau dieses Reaktanten. Ist ein vollständiger Abbau des Acetessigsäurealkylesters nicht erforderlich, so kann die Selektivität, bezogen auf den eingesetzten Alkohol, um bis zu 2 Prozentpunkte erhöht werden, wenn man den Acetessigsäureester im Überschuß einsetzt (Molverhältnis Alkohol zu Acetessigsäureester zwischen 0.7 und 0.9). In diesem Fall ist jedoch mit Einbußen bei der Selektivität bezogen auf den Acetessigester zu rechnen, so daß sich diese Vorgehensweise nur bei sehr teuren Alkoholen lohnt. Eine Rückführung der nicht umgesetzten Reaktanten ist sonst in jedem Fall sinnvoll.

Das erfindungsgemäße Verfahren läßt sich prinzipiell auf alle bekannten Varianten der sogenannten Carroll'schen Reaktion anwenden, bei denen der eingesetzte ungesättigte Alkohol unterhalb von 140°C siedet. Besondere Bedeutung besitzt das Verfahren jedoch für die Synthese des Riechstoffs 2-Methyl-2-hepten-6-on ausgehend von 2-Methyl-3-buten-2-ol.

Die Reaktion gelingt prinzipiell mit beliebigen Acetessigsäurealkylestern, jedoch werden der Methylester, der Ethylester und der Isopropylester sowohl aus wirtschaftlichen als auch aus verfahrenstechnischen Gründen bevorzugt, da die hieraus abzuspaltenden Alkanole besonders niedrig sieden und so leicht aus dem Reaktionsgemisch entfernt werden können.

Als organische Aluminiumverbindungen kommen für das erfindungsgemäße Verfahren Verbindungen der allgemeinen Formel 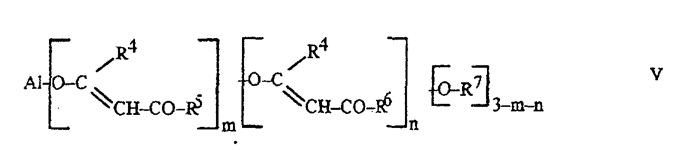 in der R⁴ Alkyl- oder Alkoxygruppen mit 1 bis 4 C-Atomen, vorzugsweise Methyl- oder Ethylgruppen bedeutet, R⁵ und R⁶ Alkyl- oder Alkoxygruppen mit 1 bis 5 C-Atomen, vorzugsweise Methyl- oder 2-Butylgruppe bedeuten, R⁷ für eine Alkylgruppe mit 1 bis 4 C-Atomen steht und m sowie n Werte von 0 bis 3 annehmen können, wobei n+m ≤ 3 ist, und Aluminiumtriaryloxylate in Betracht. Ganz besonders bevorzugt sind flüssige Aluminiumverbindungen, bei denen R⁵ ein Methylrest und R⁶ ein Butylrest, die Summe n+m = 3 und das Verhältnis n/m > 0.3 ist.

Bei den erstgenannten Katalysatoren handelt es sich also um niedere Aluminiumtrialkoholate wie Aluminiumtrimethylat, -triethylat, -triisopropylat, -tri-sek.-butylat sowie um Verbindungen, die bei der Umsetzung der genannten Aluminiumtrialkoholate mit stöchiometrischen Mengen von Acetylacetonat, Alkylacetoacetat oder Alkylmalonat unter Alkoholabspaltung und Umesterung gebildet werden. Genannt seien beispielsweise Aluminium-triacetoacetat, Aluminium-triacetylacetonat, Aluminium-monoacetoacetat-diethylat, Aluminium-monoacetoacetat-diisopropylat, Aluminium-diacetoacetatmonoisopropylat. Bevorzugt verwendet man die Aluminiumtrialkoholate, insbesondere das Aluminiumtriisopropylat sowie das Aluminium-tri-sek.-butylat. Ganz besonders bevorzugt verwendet man das gemischte Aluminiumtriacetoacetat, das man durch Umsetzung von Aluminium-sek.-butylat mit Acetessigsäuremethylester unter Abspaltung von 2-Butanol und Umesterung der Methoxygruppen mit dem freigesetzten 2-Butanol erhält, wobei der Umesterungsgrad über 30% beträgt.

Als Aluminiumtriaryloxylate bezeichnen wir die Aluminiumsalze von aromatischen Hydroxyverbindungen wie Aluminiumtriphenolat, Aluminiumtrikresolate, Aluminiumtrixylenolate, Aluminiumtrinaphtholate, deren Arylreste auch durch niedere Alkyl- oder Alkyloxygruppen, d.h. Alkyl- oder Alkyloxygruppen mit 1 bis 4 C-Atomen , Hydroxygruppen oder Phenyl substituiert sein können. Mit besonderem Vorteil verwendet man das relativ leicht zugängliche Aluminiumtriphenolat.

Die Menge der Aluminiumverbindung wird allgemein so bemessen, daß ihre Konzentration im Reaktionsgemisch 0.05 Gew.% Al nicht unterschreitet und zu Beginn der Reaktion 6 Gew.% Al nicht überschreitet. Bezogen auf umzusetzenden Acetessigsäurealkylester werden im allgemeinen 0.5 bis 5 Mol% an der Aluminiumverbindung benötigt. Für das bevorzugt verwendete Aluminiumtriisopropylat und das oben beschriebene gemischte, aus Aluminium-sek.-butylat und Acetessigsäuremethylester hergestellte Aluminiumtriacetoacetat werden z. B. Mengen von etwa 1 bis 3 Mol% bezogen auf den umzusetzenden Acetessigsäurealkylester eingesetzt.

Im allgemeinen wählt man bei dem erfindungsgemäßen Verfahren gemäß die Einsatzmengen der Reaktanten so aus, daß sich ein Molverhältnis von ungesättigtem Alkohol zu Acetessigsäurealkylester zwischen 0,7 und 1,2, vorzugsweise zwischen 0,95 und 1,05 ergibt.

Den Druck im Reaktionsgefäß kann man durch Aufpressen eines Inertgases und/oder durch Sammlung und Aufpressen des bei der Reaktion gebildeten Kohlendioxids einstellen, wobei letzteres bevorzugt wird.

Die für den Verfahrenserfolg auch sehr wesentliche Reaktionstemperatur kann man prinzipiell durch geeignete Variation des Wärmeeintrags und/oder durch Variation der Zugabegeschwindigkeit des Acetessigsäurealkylesters regeln.

Zur Erleichterung des Abdestillierens des bei der Umsetzung gebildeten Alkanols und zur Vermeidung von Siedeverzügen ist es von Vorteil für eine ausreichende Durchmischung des Reaktionsgemisches im Reaktionsgefäß zu sorgen. Dies kann man prinzipiell mit Hilfe eines starken Rührers erreichen. Mit besonderem Vorteil gelingt dies aber durch ständiges Umpumpen des Reaktionsgemisches durch einen äußeren Flüssigkeitskreislauf, durch Eintragen des Acetessigesters durch eine Mischdüse in das Reaktionsgefäß oder in den äußeren Flüssigkeitskreislauf oder aber durch Einleiten eines Inertgasstromes bzw. von rückgeführtem Kohlendioxid.

Vorteilhaft gestaltet sich das erfindungsgemäße Verfahren, wenn man den Katalysator zusammen mit als Nebenprodukt gebildeten Hochsiedern vom Reaktionsgemisch abtrennt und nach Austausch von jeweils 1 bis 40 Gew.-% , vorzugsweise zwischen 20 und 30 Gew.-%, dieser Mischung gegen frischen Katalysator austauscht und diesen in die Synthese zurückführt.

Besonders gute Selektivitäten erzielt man, wenn man nach Beendigung der Zugabe des Acetessigsäurealkylesters zur Nachreaktion den Druck im Reaktionsgefäß und damit die Reaktionstemperatur absenkt und darüberhinaus auf diese Weise die Entfernung von Restmengen an dem Alkanol der Formel IV aus dem Reaktionsgemisch erleichtert.

Das erfindungsgemäße verfahren kann sowohl diskontinuierlich als auch kontinuierlich sehr vorteilhaft durchgeführt werden.

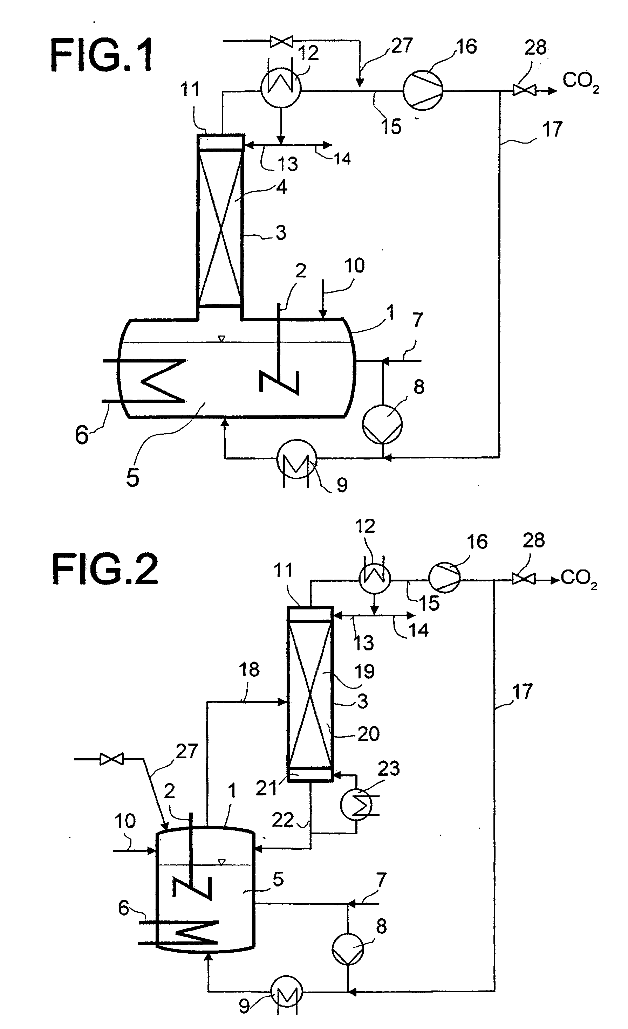

Diskontinuierliche Durchführung des erfindungsgemäßen Verfahrens (vgl. Figur 1)

Als Reaktorsystem für die diskontinuierliche Reaktionsführung eignet sich beispielsweise eine übliche beheizte Destillationsblase (1) mit Rührwerk (2) und aufgesetzter Fraktionierkolonne (3), wie sie in Figur 1 schematisch dargestellt ist. Als Fraktionierkolonne kann beispielsweise eine Kolonne mit etwa 2 bis 40 theoretischen Stufen verwendet werden. Dabei sind alle Typen von Kolonneneinbauten (4), wie strukturierte geordnete Packungen, Kolonnenböden verschiedener Bauart oder auch regellose Schüttungen von Kolonnenfüllkörpern einsetzbar. Die Temperatur im Reaktionssystem wird durch Variation von Wärmeeintrag durch die Wärmequelle (6) und ggf. (9), durch Variation des Druckes, durch Zufuhr von ggf erwärmtem Inertgas und/oder durch geeignete Variation der Dosierung des Acetessigsäurealkylesters auf dem gewünschten Niveau gehalten.

Zweckmäßigerweise wird der ungesättigte Alkohol der Formel II zusammen mit der als Katalysator verwendeten Aluminiumverbindung in der Reaktionsblase (1) vorgelegt. Anschließend wird über eine Leitung (27) ein Inertgas wie Stickstoff oder Kohlendioxid aufgepresst und dadurch der gewünschte Reaktionsdruck eingestellt. Es ist ebenfalls möglich, den Druck zunächst nur soweit zu erhöhen, daß die Siedetemperatur des vorgelegten Alkohols hoch genug für ein Anspringen der Carroll-Reaktion ist, und anschließend mit Hilfe des während der Reaktion entstehenden Kohlendioxids den gewünschten Systemdruck aufzubauen. In der Regel sollte die Anfangssiedetemperatur des Reaktionsgemisches bei mindestens 110°C liegen.

Danach wird der Blaseninhalt (5) mit einem Heizregister (6) oder mit einem im äußeren Kreislauf (7) mit Pumpe (8) installierten Wärmetauscher (9) bei unendlichem Rücklaufverhältnis aufgeheizt. Nach Einstellen der gewünschten Reaktionstemperatur wird der Acetessigsäurealkylester der Formel (III) über einen Zulauf (10) so in die Blase (1) oder den äußeren Kreislauf (7) zudosiert, daß ein konstanter Gehalt des Acetessigsäurealkylesters zwischen 0.5 und 10 Gew.-%, vorzugsweise zwischen 1 und 3 Gew.-%, in der Reaktionslösung (5) erreicht wird. Die Einstellung des Drucks während der Reaktion erfolgt mit Hilfe einer Druckregelung (28).

Mit dem Beginn der Dosierung des Acetessigsäurealkylesters setzt die Bildung von Alkanol der allgemeinen Formel R³-OH (IV) und Kohlendioxid ein. Der sich zu Beginn am Kopf der Kolonne befindende ungesättigte Alkohol der Formel (II) wird durch das freiwerdende Alkanol der Formel (IV) verdrängt. Das Rücklaufverhältnis wird auf einen geeigneten Wert eingestellt, und die Reaktionsprodukte werden mit Hilfe der aufgesetzten Kolonne (3) über den Kopf (11) der Kolonne abgetrennt und in den Kondensator (12) geleitet. Das auskondensierte Alkanol R³-OH wird zum einen Teil als Rücklauf (13) auf die Kolonne (3) zurückgeführt und zum anderen Teil (14) als Destillat abgezogen. Das den Kondensator verlassende Kohlendioxid (15) kann mit Hilfe eines Kompressors (16) aus den genannten Gründen über die Leitung (17) in die Reaktionsblase (1) zurückgeführt werden. Die Dosierung des Acetessigsäurealkylesters über den Zulauf (10) dauert im allgemeinen ca. 2 bis 4 Stunden. Wenn quantitativer Umsatz des Acetessigsäurealkylesters erwünscht ist, sollte das Reaktionsgemisch nach Beendigung des Zulaufs noch etwa 1 bis 2 Stunden bei der Reaktionstemperatur gehalten werden. Es ist jedoch von Vorteil, nach Beendigung der Zudosierung des Acetessigsäurealkylesters, in der sogenannten Nachreaktionsphase, den Druck langsam auf Normaldruck abzusenken, da hierdurch die vollständige Abtrennung des gebildeten Alkanols erleichtert wird. Die Reaktionstemperatur in der Nachreaktionsphase sollte 180°C nicht überschreiten.

Das Fortschreiten der Reaktion kann durch Messung der Kohlendioxidentwicklung in Leitung (15) und/oder anhand der Menge des aus dem Acetessigsäurealkylester abgespaltenen Alkohols (14) verfolgt werden. Die Konzentration des Acetessigsäurealkylesters im Reaktionsgemisch (5) kann durch gaschromatografische Analyse bestimmt werden.

Alternative diskontinuierliche Verfahrensführung gemäß Figur 2.

Als Variante des oben beschriebenen Verfahrens ist es auch möglich, als Reaktor eine Reaktionsblase (1) mit nicht direkt aufgesetzter Fraktionierkolonne (3) zu verwenden. Auch hierbei ist die Reaktionsblase gegebenenfalls mit einem Rührer (2) und einem Heizregister als Wärmequelle (6) und ggf. mit einem äußeren Kreislauf (7) enthaltend eine Pumpe (8) und ggf. eine Wärmequelle (9) ausgestattet. Die Zudosierung des Acetessigsäurealkylesters erfolgt durch den Zulauf (10). Die aus der Reaktionsblase (1) kommenden Brüden (18) werden in eine Kolonne (3) mit Verstärkungsteil (19) und Abtriebsteil (20) eingeleitet. Dabei wird das sich bildende Kohlendioxid zusammen mit dem entstehenden Alkanol über den Kopf (11) der Kolonne abgetrennt und in den Kondensator (12) geleitet. Analog zur oben beschriebenen Variante wird der Alkohol R³OH im Kondensor (12) kondensiert und teilweise als Rücklauf (13) auf die Kolonne zurückgeführt. Ebenso kann das Kohlendioxid mit einem Kompressor (16) komprimiert und über die Leitung (17) in die Reaktionsblase (1) zurückgeführt werden. Der nicht umgesetzte Reaktanten enthaltende Sumpfablauf (22) der Kolonne (3) wird in die Reaktionsblase (1) zurückgeführt. Die Dosierung und Reaktionsführung entsprechen dem oben beschriebenen Verfahren.

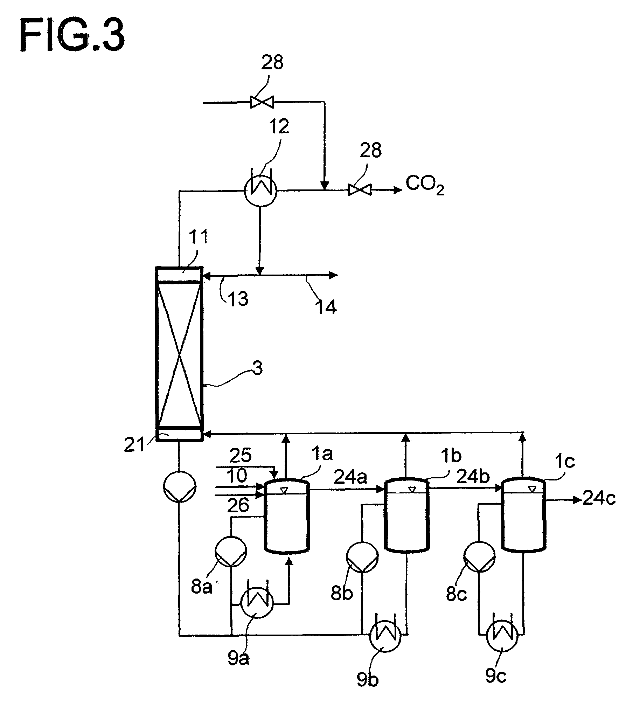

### Kontinuierliche Reaktionsführung (vgl. Figur 3)

Bei kontinuierlicher Reaktionsführung kann als Reaktorsystem beispielsweise eine beheizte Kesselkaskade mit 1 bis 10, zweckmäßigerweise 2 bis 4 Kesseln, verwendet werden. Hierbei sind die einzelnen Kessel (z.B.1a-1c) je durch einen Überlauf (z.B. 24a-24c) miteinander verbunden. Es kann jedem Kessel eine separate Kolonne aufgesetzt sein oder aber - wie in Figur 3 dargestellt - nur eine Kolonne (3) für alle Kessel.

Die Reaktanten werden kontinuierlich in den ersten Kessel (1a) eingebracht und zwar die Alkohole der allgemeinen Formel II über den Zulauf (25), der Acetessigsäurealkylester über den Zulauf (10) sowie gegebenenfalls der Aluminiumkatalysator über den Zulauf (26). Das Einstellen des notwendigen Reaktionsdruckes in der Reaktionsblase (1) sowie der hiermit verbundenen Kolonne mit einem externen Inertgas und/oder dem gebildeten Kohlendioxid erfolgt durch eine Druckregeleinrichtung (28) vorteilhaft auf die in Figur 3 schematisch dargestelte Weise. Für das Einhalten der gewünschten Reaktionstemperatur und die Arbeitsweise der Kolonne (3) gilt im wesentlichen das gleiche wie für die diskontinuierliche Fahrweise.

Weiterhin ist es möglich, den verwendeten Katalysator nach Beendigung der Reaktion z.B. mit Hilfe eines Dünnschichtverdampfers abzutrennen und in die Synthese zurückzuführen. Dabei ist es von Vorteil, jeweils 1 bis 40 Gew.-% des Rückstandes einer Charge, bevorzugt zwischen 20 und 30 Gew.-%, gegen frischen Katalysator auszutauschen und auf diese Weise gleichzeitig die Hochsieder auszuschleusen.

Mit Hilfe des erfindungsgemäß verbesserten Verfahrens ist es möglich, zahlreiche Ketone, insbesondere das 2-Methyl-2-hepten-6-on, bei nahezu quantitativem Umsatz in sehr hohen Ausbeuten und Raumzeitausbeuten sowie hoher Reinheit herzustellen.

### Beispiel 1:

### Herstellung von 2-Methyl-2-hepten-6-on bei 1.7 bis 4 bar

Die Versuchsapparatur bestand aus einem beheizbaren, mit Rührer ausgestatteten 2-Liter-Reaktionskolben aus Edelstahl, auf den eine Destillationskolonne (Länge: lm, Durchmesser: 25 mm) aufgesetzt war. Die Kolonne war mit Edelstahldrahtwendeln (Durchmesser: 5 mm) gefüllt. Die Reaktanten wurden mit Hilfe einer Pumpe aus den Vorlagebehältern, die auf einer Waage standen, zudosiert. Die während der Reaktion freigesetzten Komponenten Methanol und CO₂, sowie die flüchtigen Nebenprodukte Isopren und Aceton wurden über die Kolonne abgetrennt und in einem Teilkondensator kondensiert. Das Kondensat lief über einen Rücklaufteiler in einen Vorlagebehälter. Der verbleibende Abgasstrom wurde durch eine Kühlfalle und anschließend zur Volumenmessung durch eine Gasuhr geleitet. Die Apparatur war mit einer Druckregelung ausgestattet und auf einen Systemdruck von 10 bar ausgelegt. Alle ein- und austretenden Stoffströme wurden während des gesamten Versuchs kontinuierlich erfaßt und registriert, so daß eine zeitabhängige Massenbilanzierung möglich war.

25.0 g (0,12 mol) Aluminiumtriisopropylat, entsprechend 1.7 Mol-%, bezogen auf die Gesamtmenge an Acetessigsäuremethylester (AME), wurden in einem 200 ml Rührkolben mit 87.0 g (0.75 mol) AME vermischt und das Gemisch auf 140°C erwärmt. Hierbei löste sich das Aluminiumtriisopropylat im AME auf. Danach wurden 680.0 g 2-Methyl-3-buten-2-ol (MBE; 93%ig; 7.34 mol berechnet auf 100%) in dem 2-Liter-Rührautoklaven mit aufgesetzter Kolonne vorgelegt und auf 90°C aufgeheizt. Die 140°C heiße Katalysatorlösung wurde anschließend mit dem 90°C heißen MBE im Rührautoklaven vermischt. Anschließend wurde mit einer Gasflasche Stickstoff aufgepresst und hierüber ein Reaktordruck von 1.7 bar (abs.) eingestellt. Anschließend wurde das Reaktionsgemisch bei unendlichem Rücklaufverhältnis mit dem Thermostaten auf 120°C aufgeheizt. Ab einer Temperatur von 120°C wurden 724.3 g (6.24 mol) AME innerhalb von 180 Minuten (min) linear in den Rührautoklaven dosiert. Mit dem Beginn der Dosierung sprang die Reaktion an, so daß die CO₂-Entwicklung einsetzte. Hierdurch wurde innerhalb von 30 min ein Reaktionsdruck von 4 bar (abs.) aufgebaut. Der Druck wurde anschließend über die Druckregelung aufrechterhalten.

In der Anfahrphase destillierte überwiegend MBE in die Kolonne, das bei totalem Rücklauf in den Rührautoklaven zurückgeführt wurde. Das MBE wurde mit Beginn der Dosierung durch das während der Reaktion freigesetzte Methanol verdrängt. Sobald eine Kopftemperatur von 104 °C (Siedetemperatur von Methanol bei 4 bar) erreicht war, wurde das Rücklaufverhältnis (RV) auf 14 eingestellt. Das entstandene Methanol wurde über die aufgesetzte Kolonne als Destillat abgetrennt und das CO₂ mit Hilfe der Druckregelung so abgeführt, dass der Reaktordruck auf 4 bar gehalten wurde. Die Temperatur (T) des Reaktionsgemisches stieg während der Reaktion auf 185 °C an und wurde danach auf 185 °C geregelt. Nach 180 min war die AME-Dosierung abgeschlossen. Mit Abschluß der AME-Dosierung begann die Nachreaktion. Die Apparatur wurde langsam von 4 bar auf Normaldruck entspannt, wobei die Temperatur (T) von 185 °C auf 170 °C absank. T wurde anschließend auf 170 °C geregelt. Die Nachreaktion wurde nach 90 min beendet. Der Katalysator und die Hochsieder wurden durch einstufige Destillation abgetrennt und das Destillat gaschromatographisch analysiert.

2-Methyl-2-hepten-6-on wurde bei einem MBE-Umsatz von 88,7% und einem nahezu quantitativen AME-Umsatz in einer Selektivität von 92.1 % der Theorie (d. Th.), bezogen auf umgesetztes MBE und von 86,1% d. Th., bezogen auf umgesetztes AME erhalten.

### Beispiel 2

### Herstellung von 2-Methyl-2-hepten-6-on bei 4 bar

25.0 g (0,12 mol) Aluminiumtriisopropylat, entsprechend 1.7 Mol-% bezogen auf die Gesamtmenge an AME, wurden in einem 200ml Rührkolben mit 87.0 g (0.75 mol) AME vermischt und auf 140°C aufgeheizt. Hierbei löste sich das Alumimiumtriisopropylat im AME auf. Danach wurden in der unter Beispiel 1 beschriebenen Versuchsapparatur 672.0 g MBE (94%-ig; 7.33 mol berechnet auf 100%) in dem 2-Liter-Rührautoklaven mit aufgesetzter Kolonne vorgelegt und auf 100°C aufgeheizt. Die 140°C heiße Katalysatorlösung wurde anschließend mit dem 100°C heißen MBE im Rührautoklaven vermischt. Danach wurde mit einer Gasflasche Stickstoff aufgepresst und hierüber ein Reaktordruck von 4.0 bar absolut (abs.) eingestellt. Anschließend wurde das Reaktionsgemisch bei unendlichem Rücklaufverhältnis mit dem Thermostaten auf 145°C aufgeheizt und 724.3 g (6.24 mol) AME linear innerhalb von 180 min in den Rührautoklaven dosiert. Mit dem Beginn der Dosierung sprang die Reaktion an, so daß die CO₂-Entwicklung einsetzte. Der Druck wurde über die Druckregelung aufrechterhalten. Sobald eine Kopftemperatur von 104 °C (Siedetemperatur von Methanol bei 4 bar) erreicht wurde, wurde das Rücklaufverhältnis RV auf 15 eingestellt. Das entstandene Methanol wurde über die aufgesetzte Kolonne als Destillat abgetrennt und das CO₂ mit Hilfe der Druckregelung so abgeführt, dass der Reaktordruck auf 4 bar gehalten wurde. Die T des Reaktionsgemisches stieg während der Reaktion auf 185 °C an und wurde danach auf 185 °C geregelt. Nach 180 min war die AME-Dosierung abgeschlossen. Mit Abschluß der AME-Dosierung begann die Nachreaktion, in der die Apparatur noch 15 min auf 4 bar gehalten wurde. Danach wurde langsam von 4 bar auf Normaldruck entspannt, wobei T von 185 °C auf 170 °C absank. Die T wurde anschließend auf 170 °C geregelt. Die Nachreaktion wurde nach 90 min beendet. Der Katalysator und die Hochsieder wurden durch einstufige Destillation abgetrennt und das Destillat gaschromatographisch analysiert.

Das 2-Methyl-2-hepten-6-on wurde bei einem MBE-Umsatz von 87 % und einem nahezu quantitativen AME-Umsatz in einer Selektivität von 90.7 % d.Th., bezogen auf umgesetztes MBE, und einer Selektivität von 83% d. Th., bezogen auf umgesetztes AME, erhalten.

### Beispiel 3

### Herstellung von 2-Methyl-2-hepten-6-on bei 4 bar unter Rückführung des Katalysators

Eine Versuchsreihe unter Rückführung des Katalysators mit 11 Rückführungszyklen wurde analog zu Beispiel 1 in der oben beschriebenen Apparatur durchgeführt. Hierbei wurde nach jedem Versuch das gesamte Reaktionsgemisch durch einstufige Destillation aufgearbeitet und jeweils 25 Gew.-% des angefallenen Rückstands ausgeschleust. Dem verbleibenden Rückstand wurde die Menge an frischem Katalysator zugefügt, die in der ausgeschleusten Masse enthalten war. Die entsprechende Menge an frischem Al(OiPr)₃ wurde direkt im Rückstand aufgelöst und bei dem folgenden Versuch als Katalysator eingesetzt. Bei dieser Vorgehensweise wurde über 11 Versuche bei einem durchschnittlichen MBE-Umsatz von 88 % und einem nahezu quantitativen AME-Umsatz eine Selektivität von durchschnittlich 90 % d.Th., bezogen auf umgesetztes MBE, und eine Selektivität von durchschnittlich 83 % d.Th., bezogen auf umgesetztes AME, erzielt. Ein Abfallen der Ausbeuten war auch nach zehn Rückführungen nicht zu beobachten.

## Patentansprüche

1. Verfahren zur Herstellung von ungesättigten Ketonen der allgemeinen Formel I 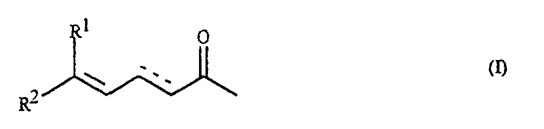 in der die gestrichelte Linie eine zusätzliche C-C-Bindung bedeuten kann, R¹ für eine Alkylgruppe mit 1 oder 2 C-Atomen steht und R² für eine Alkylgruppe mit 1 bis 4 C-Atomen steht, durch Umsetzen von α,β-ungesättigten Alkoholen der allgemeinen Formel II 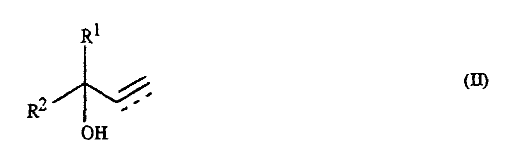 mit Acetessigsäurealkylestern der allgemeinen Formel III 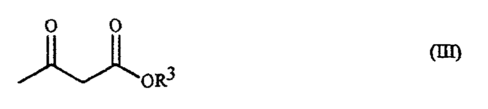 in der R³ für eine Alkylgruppe mit 1 bis 4 C-Atomen steht, in Gegenwart von 0.1 bis 5 Mol-%, bezogen auf den umzusetzenden Acetessigsäurealkylester, an einer organischen Aluminiumverbindung als Katalysator unter Abspaltung und fortlaufender destillativer Entfernung des sich während der Reaktion aus dem Acetessigsäurealkylester abspaltenden Alkohols der allgemeinen Formel IV
R³-OH (IV)
in einem Reaktorsystem mit aufgesetzter Fraktionierkolonne, **dadurch gekennzeichnet, daß** man
A einen α,β-ungesättigten Alkohol, der unterhalb von 140°C siedet, ohne Mitverwendung wirksamer Mengen eines Lösungsmittels zusammen mit der organischen Aluminiumverbindung im Reaktionsgefäß vorlegt,
B unter erhöhtem Druck, eine möglichst konstante Reaktionstemperatur zwischen 170°C und 250°C einstellt,
C bei dieser Temperatur zu dem gemäß A erhaltenen Gemisch aus dem α,β-ungesättigten Alkohol und der organischen Aluminiumverbindung den Acetessigsäurealkylester zudosiert und
D während der Umsetzung den Gehalt an dem Acetessigsäurealkylester im Reaktionsgemisch auf einen möglichst konstanten Wert zwischen 0.1 und 10 Gew.-% einstellt.

2. Verfahren gemäß Anspruch 1, **dadurch gekennzeichnet, daß** man als organische Aluminiumverbindung eine Aluminiumverbindung der allgemeinen Formel (V) 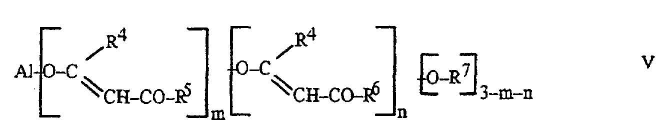 in der R⁴ Alkyl- oder Alkoxygruppen mit 1 bis 4 C-Atomen, vorzugsweise Methyl- oder Ethylgruppen bedeutet, R⁵ und R⁶ Alkyl- oder Alkoxygruppen mit 1 bis 5 C-Atomen, vorzugsweise Methyl- oder eine 2-Butylgruppe bedeuten, R⁷ für eine Alkylgruppe mit 1 bis 4 C-Atomen steht und m sowie n Werte von 0 bis 3 annehmen können, wobei n+m ≤ 3 ist, oder ein Aluminiumtriaryloxylat verwendet.

3. Verfahren gemäß Anspruch 1, **dadurch gekennzeichnet, daß** man die Einsatzmengen der Reaktanten so wählt, daß sich ein Molverhältnis von Alkohol zu Acetessigsäurealkylester zwischen 0,95 und 1,05 ergibt.

4. Verfahren gemäß Anspruch 1, **dadurch gekennzeichnet, daß** man als α,β-ungesättigten Alkohol der allgemeinen Formel II, der unter Normalbedingungen unterhalb von 140°C siedet, 2-Methyl-3-buten-2-ol verwendet.

5. Verfahren gemäß Anspruch 4, **dadurch gekennzeichnet, daß** man den Druck im Reaktionsgefäß durch Aufpressen eines Inertgases und/oder durch Sammlung und Aufpressen des bei der Reaktion gebildeten Kohlendioxids einstellt.

6. Verfahren gemäß Anspruch 1, **dadurch gekennzeichnet, daß** man die Reaktionstemperatur durch geeignete Variation des Wärmeeintrags und/oder durch Variation der Zugabegeschwindigkeit des Acetessigsäurealkylesters regelt.

7. Verfahren gemäß Anspruch 1, **dadurch gekennzeichnet, daß** man mit Hilfe eines Rührers, durch Umpumpen des Reaktionsgemisches durch einen äußeren Flüssigkeitskreislauf, durch Eintragen des Acetessigsäurealkylesters durch eine Mischdüse oder aber durch Einleiten eines Inertgasstromes oder von rückgeführtem Kohlendioxid für eine ausreichende Durchmischung des Reaktionsgemisches im Reaktionsgefäß sorgt und damit das Abdestillieren des gebildeten Alkanols der allgemeinen Formel IV erleichtert.

8. Verfahren gemäß Anspruch 1, **dadurch gekennzeichnet, daß** man den Katalysator zusammen mit als Nebenprodukt gebildeten Hochsiedern vom Reaktionsgemisch abtrennt und nach Austausch von jeweils 1 bis 40 Gew.% gegen frischen Katalysator in die Synthese zurückführt.

9. Verfahren gemäß Anspruch 1, **dadurch gekennzeichnet, daß** man nach Beendigung der Zugabe des Acetessigsäurealkylesters zur Nachreaktion den Druck im Reaktionsgefäß absenkt.

## Claims

1. A process for preparing unsaturated ketones of the general formula I 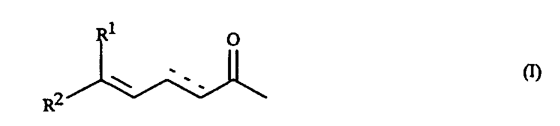 in which the dotted line can be an additional C-C bond, R¹ is an alkyl group with 1 or 2 C atoms, and R² is an alkyl group with 1 to 4 C atoms, by reacting α,β-unsaturated alcohols of the general formula II 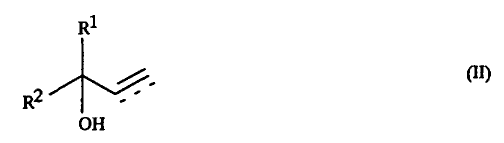 with alkyl acetoacetates of the general formula III 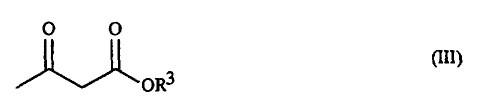 in which R³ is an alkyl group with 1 to 4 C atoms, in the presence of from 0.1 to 5 mol%, based on the alkyl acetoacetate to be reacted, of an organic aluminum compound as catalyst with elimination and continuous removal by distillation of the alcohol which is eliminated from the alkyl acetoacetate during the reaction and has the general formula IV
R³-OH (IV)
in a reactor system with a fitted fractionation column, wherein
A an α,β-unsaturated alcohol which boils below 140°C is introduced, in the absence of effective amounts of a solvent, together with the organic aluminum compound into the reaction vessel,
B a reaction temperature which is as constant as possible between 170°C and 250°C is set under elevated pressure,
C at this temperature, the alkyl acetoacetate is metered into the mixture, obtained in A, of the α,β-unsaturated alcohol and the organic aluminum compound, and
D during the reaction the content of alkyl acetoacetate in the reaction mixture is set at a value which is as constant as possible between 0.1 and 10% by weight..

2. A process as claimed in claim 1, wherein the organic aluminum compound used is an aluminum compound of the general formula (V) 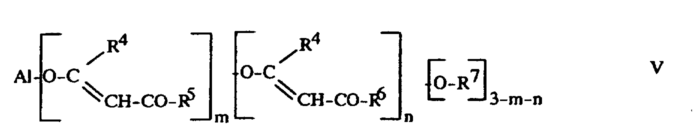 in which R⁴ are alkyl or alkoxy groups with 1 to 4 C atoms, preferably methyl or ethyl groups, R⁵ and R⁶ are alkyl or alkoxy groups with 1 to 5 C atoms, preferably methyl or a 2-butyl group, R⁷ is an alkyl group with 1 to 4 C atoms, and m and n can assume values from 0 to 3, where n+m ≤ 3, or an aluminum triaryloxide.

3. A process as claimed in claim 1, wherein the amounts of the reactants employed are chosen to result in a molar ratio of alcohol to alkyl acetoacetate of between 0.95 and 1.05.

4. A process as claimed in claim 1, wherein 2-methyl-3-buten-2-ol is used as α,β-unsaturated alcohol of the general formula II which boils below 140°C under normal conditions.

5. A process as claimed in claim 4, wherein the pressure in the reaction vessel is set by injecting an inert gas and/or by collecting and injecting the carbon dioxide formed in the reaction.

6. A process as claimed in claim 1, wherein the reaction temperature is controlled by suitable variation of the heat input and/or by variation of the rate of addition of alkyl acetoacetate.

7. A process as claimed in claim 1, wherein adequate mixing of the reaction mixture in the reaction vessel is ensured by use of a stirrer, by pumping the reaction mixture through an external liquid circulation, by introducing the alkyl acetoacetate through a mixing nozzle or else by passing in a stream of inert gas or recycled carbon dioxide, and thus the distillative removal of the alkanol of the general formula IV which is formed is facilitated.

8. A process as claimed in claim 1, wherein the catalyst is removed, together with high boilers formed as byproducts, from the reaction mixture and, after replacement of in each case 1 to 40% by weight with fresh catalyst, is returned to the synthesis.

9. A process as claimed in claim 1, wherein after completion of the addition of the alkyl acetoacetate the pressure in the reaction vessel is lowered for after-reaction.

## Revendications

1. Procédé pour la préparation de cétones insaturées de formule générale I 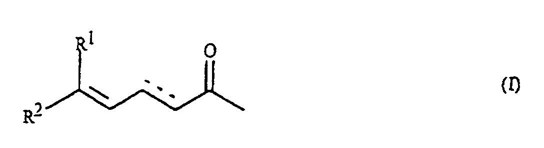 dans laquelle la ligne pointillée peut représenter une liaison C-C additionnelle, R¹ représente un groupe alkyle ayant 1 ou 2 atomes de carbone, et R² représente un groupe alkyle ayant 1 à 4 atomes de carbone, par réaction d'alcools α,β-insaturés de formule générale II 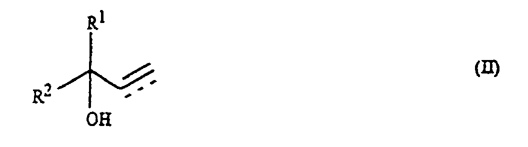 avec des esters alkyliques d'acide acétylacétique de formule générale III 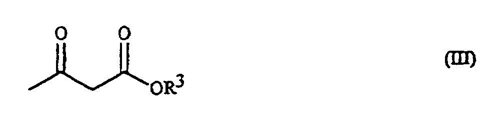 dans laquelle R³ désigne un groupe alkyle ayant 1 à 4 atomes de carbone, en présence de 0,1 à 5 % en mol, par rapport à l'ester alkylique d'acide acétylacétique à faire réagir, d'un composé organique d'aluminium en tant que catalyseur avec séparation et élimination en continu par distillation de l'alcool, de formule générale IV, qui se sépare de l'ester alkylique de l'acide acétylacétique pendant la réaction
R³-OH (IV)
dans un système de réacteur dans lequel est installée une colonne de fractionnement, **caractérisé par le fait qu'**on
A introduit un alcool α,β-insaturé bouillant en dessous de 140°C, sans utiliser conjointement de quantités actives d'un solvant, avec le composé organique d'aluminium dans le récipient de réaction,
B sous pression élevée, on établit une température de réaction la plus constante possible entre 170°C et 250°C,
C à cette température on ajoute en quantité dosée, au mélange obtenu selon A de l'alcool α,β-insaturé et du composé organique d'aluminium, l'ester alkylique d'acide acétylacétique et
D pendant la réaction on ajuste la teneur en ester alkylique d'acide acétylacétique dans le mélange réactionnel à une valeur la plus constante possible entre 0,1 et 10 % en poids.

2. Procédé selon la revendication 1, **caractérisé par le fait qu'**on utilise comme composé organique d'aluminium un composé d'aluminium de formule générale (V) 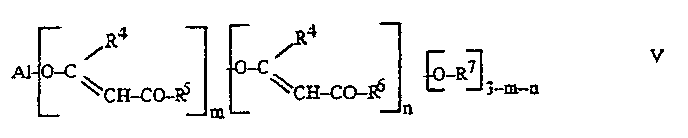 dans laquelle R⁴ désigne des groupes alkyle ou alcoxy ayant 1 à 4 atomes de carbone, de préférence des groupes méthyle ou éthyle, R⁵ et R⁶ représentent des groupes alkyle ou alcoxy ayant 1 à 5 atomes de carbone, de préférence un groupe méthyle ou 2-butyle, R⁷ désigne un; groupe alkyle ayant 1 à 4 atomes de carbone, et m ainsi que n peuvent prendre des valeurs de 0 à 3, tandis que n+m ≤ 3, ou un triaryloxylate d'aluminium.

3. Procédé selon la revendication 1, **caractérisé par le fait qu'**on choisit la quantité introduite des corps réagissants de façon à avoir en rapport molaire de l'alcool à l'ester alkylique d'acide acétylacétique entre 0,95 et 1,05.

4. Procédé selon la revendication 1, **caractérisé par le fait qu'**on utilise comme alcool α,β-insaturé de formule générale II bouillant en dessous de 140°C dans les conditions normales, le 2-méthyl-3-butène-2-ol.

5. Procédé selon la revendication 4, **caractérisé par le fait qu'**on ajuste la pression dans le récipient de réaction par compression d'un gaz inerte et/ou par recueil et compression du dioxyde de carbone formé dans la réaction.

6. Procédé selon la revendication 1, **caractérisé par le fait qu'**on règle la température de réaction par variation appropriée de l'apport de chaleur et/ou par variation de la vitesse d'introduction de l'ester alkylique d'acide acétylacétique.

7. Procédé selon la revendication 1, **caractérisé par le fait qu'**on veille à un brassage suffisant du mélange réactionnel dans le récipient de réaction par recyclage par pompage du mélange réactionnel au moyen d'un circuit de liquide externe, par introduction de l'ester alkylique d'acide acétylacétique par l'intermédiaire d'une buse mélangeuse ou encore par introduction d'un courant de gaz inerte ou de dioxyde de carbone recyclé, et on facilite ainsi la séparation par distillation de l'alcool formé de formule générale IV.

8. Procédé selon la revendication 1, **caractérisé par le fait qu'**on sépare le catalyseur du mélange réactionnel conjointement avec les produits à point d'ébullition élevé formés en tant que sous-produit, et on le recycle dans la synthèse après remplacement à chaque fois de 1 à 40 % en poids par du catalyseur frais.

9. Procédé selon la revendication 1, **caractérisé par le fait qu'**on diminue la pression dans le récipient de réaction après achèvement de l'addition d'ester alkylique d'acide acétylacétique pour réaction subséquente.
